# EUROPEAN PATENT SPECIFICATION

(11) **EP 2 603 266 B1**
(45) Date of publication and mention of the grant of the patent: **24.05.2017**
(21) Application number: 11761137.6
(22) Date of filing: 11.08.2011
(51) Int. Cl.: A61M 16/00, A61M 16/08, A61M 16/20

(54) **SYSTEM FOR PROVIDING A PRESSURIZED FLOW OF BREATHABLE GAS TO THE AIRWAY OF A SUBJECT WITH STOCHASTIC FLUCTUATIONS**
SYSTEM FÜR DIE ZUFÜHRUNG EINES UNTER DRUCK STEHENDEN STROMS EINES ATEMGASES IN DIE ATEMWEGE EINER PERSON MIT STOCHASTISCHEN FLUKTUATIONEN
SYSTÈME PERMETTANT D'AMENER UN FLUX DE GAZ RESPIRATOIRES SOUS PRESSION AUX VOIES RESPIRATOIRES D'UN SUJET AVEC DES FLUCTUATIONS STOCHASTIQUES

(30) Priority: 13.08.2010 US 373354 P
(43) Date of publication of application: 19.06.2013
(73) Proprietor: Koninklijke Philips N.V., 5656 AE Eindhoven (NL)
(72) Inventor: GARDE, Smita, 5656 AE Eindhoven (NL); ARCILLA, Mabini, 5656 AE Eindhoven (NL); AHMAD, Samir, 5656 AE Eindhoven (NL); MADISON, Michael Edward, 5656 AE Eindhoven (NL)
(74) Representative: van Velzen, Maaike Mathilde
(86) International application number: PCT/IB2011/053581
(87) International publication number: WO 2012/020387

(56) References cited:
- EP-A2- 1 179 354
- WO-A1-2009/126739
- US-A- 4 592 349
- US-A- 5 165 398
- US-A1- 2005 051 174
- US-A1- 2008 066 754
- US-B1- 6 257 234
- US-B1- 6 708 690

## Description

### BACKGROUND OF THE INVENTION

### Field of the Invention

The invention relates to creating stochastic fluctuations in pressure at or near the airway of a subject receiving a pressurized flow of breathable gas that mimic the oscillations in pressure associated with bubble continuous positive airway pressure therapy ("bubble CPAP").

### Description of the Related Art

Conventional systems for bubble CPAP are known. Such systems are used to treat, for example, acute respiratory distress syndrome in neonatal patients. In these systems, a therapeutic pressure level is achieved at the airway of the patient by delivery of a pressurized flow of breathable gas to the airway of the patient through a respiratory circuit that includes a cannula or endotracheal tube, and submerging an expiratory limb of the respiratory circuit in a water container. As the flow of gas through the expiratory limb generates bubbles in the water (or other fluid), pressure oscillations are created that are coupled back to the airway of the patient. These relatively small, stochastic oscillations have been found therapeutically beneficial in maintaining the openness of the airway while the patient breaths. Document US4592349 discloses a ventilator for use with a source of gas under pressure for supplying such gas to the airway of a patient having an inlet adapted to be connected to the source of gas, and an outlet adapted to be connected to the airway of the patient. Document WO2009126739 discloses a pressure regulating breathing assistance apparatus which can produce pressure oscillations having high amplitudes, a low broad-band frequency spectrum and long time duration. Document US5165398 discloses a ventilator in combination with a pneumatic oscillator cartridge comprising a body with an inlet, an outlet, a diaphragm operated valve, and a servo port. Document US6257234 discloses a ventilation system which is controlled by detecting the resistance and elastance of the patient's respiratory system and adjusting the flow supplied by the ventilator accordingly. Document US2005051174 discloses an insufflation-exsufflation system with percussive assist for removal of broncho-pulmonary secretions. Document EP1179354 discloses a ventilator, comprising an inspiratory unit and an expiratory valve for regulating a flow of breathing gas and a control unit for controlling the inspiratory unit and the expiratory valve. Document US2008066754 discloses a continuous high-frequency oscillation breathing device that delivers therapy during both inhalation and exhalation in order to assist in clearing secretions from the lungs. Document US6708690 discloses a high frequency pressure oscillation device and method of providing high frequency pressure oscillations to a patient.

### SUMMARY OF THE INVENTION

The invention relates to a system configured to provide a pressurized flow of breathable gas to the airway of a subject as defined by claim 1. Advantageous embodiments are provided in the dependent claims.

One aspect of the disclosure relates to a system configured to provide a pressurized flow of breathable gas to the airway of a subject. The system comprises a pressure generator, a subject interface circuit, and a pressure fluctuation mechanism. The pressure generator is configured to generate the pressurized flow of breathable gas such that one or more gas parameters of the pressurized flow of breathable gas provide a therapeutic benefit to the subject. The subject interface circuit is configured to deliver the pressurized flow of breathable gas from the pressure generator to the airway of the subject. The pressure fluctuation mechanism is configured to create stochastic fluctuations in pressure of the pressurized flow of breathable gas at or near the airway of the subject.

Another aspect of the disclosure relates to a method of providing a pressurized flow of breathable gas to the airway of a subject. The method comprises generating the pressurized flow of breathable gas such that one or more gas parameters of the pressurized flow of breathable gas provide a therapeutic benefit to the subject; delivering the pressurized flow of breathable gas to the airway of the subject; and operating a pressure fluctuation mechanism configured to create stochastic fluctuations in pressure of the pressurized flow of breathable gas at or near the airway of the subject.

Yet another aspect of the disclosure relates to a system configured to provide a pressurized flow of breathable gas to the airway of a subject. The system comprises means for generating the pressurized flow of breathable gas such that one or more gas parameters of the pressurized flow of breathable gas provide a therapeutic benefit to the subject; means for delivering the pressurized flow of breathable gas to the airway of the subject; and means for creating stochastic fluctuations in pressure of the pressurized flow of breathable gas at or near the airway of the subject.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. In one embodiment of the invention, the structural components illustrated herein are drawn to scale. It is to be expressly understood, however, that the drawings are for the purpose of illustration and description only and are not a limitation of the invention. In addition, it should be appreciated that structural features shown or described in any one embodiment herein can be used in other embodiments as well. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.

### BRIEF DESCRIPTION OF THE DRAWINGS

FIG. 1 illustrates a system configured to deliver a pressurized flow of breathable gas to the airway of a subject, according to one or more embodiments of the invention.
FIG. 2 illustrates a plot of pressure versus time for a pressurized flow of breathable gas generated by a bubble CPAP system.
FIG. 3 illustrates a plot of pressure versus time for a pressurized flow of breathable gas generated by a pressure therapy system, in accordance with one or more embodiments of the invention.
FIG. 4 illustrates a plot of pressure versus time for a pressurized flow of breathable gas generated by a pressure therapy system, according to one or more embodiments of the invention.
FIG. 5 illustrates a system configured to deliver a pressurized flow of breathable gas to the airway of a subject, according to one or more embodiments of the invention.
FIG. 6 illustrates a pressure fluctuation valve, in accordance with one or more embodiments of the invention.
FIG. 7 illustrates a pressure fluctuation valve, according to one or more embodiments of the invention.
FIG. 8 illustrates a method of delivering a pressurized flow of breathable gas to the airway of a subject.

### DETAILED DESCRIPTION OF THE EXEMPLARY EMBODIMENT

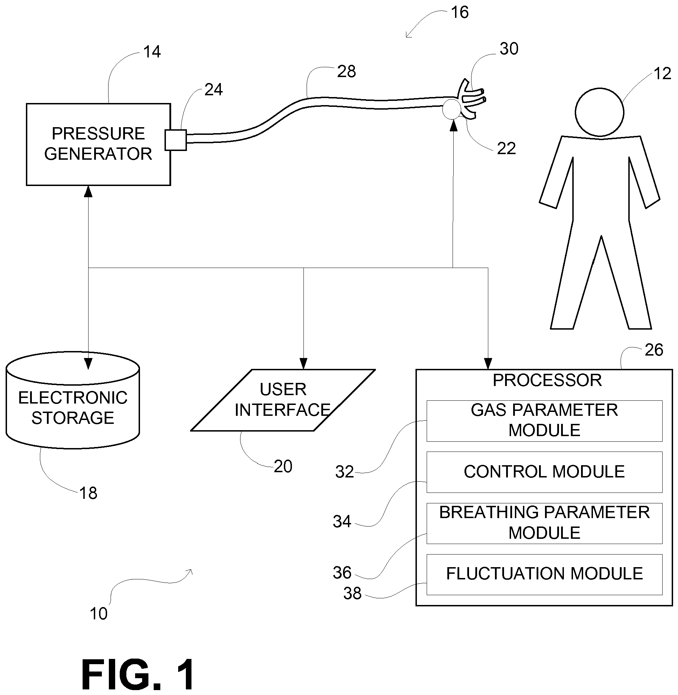

FIG. 1 illustrates a system 10 configured to deliver a pressurized flow of breathable gas to the airway of a subject 12. The system 10 provides pressurized flow of breathable gas to the airway of subject 12 such that one or more gas parameters of the pressurized flow of breathable gas provide therapeutic benefit to subject 12. In one embodiment, system 10 is configured such that the pressurized flow of breathable gas supports the airway of subject 12 to permit subject 12 to breathe. In one embodiment, system 10 is configured such that the respiration of subject 12 is mechanically assisted by the pressurized flow of breathable gas. To enhance the effectiveness in facilitating respiration by subject 12, system 10 is configured to vary the level of pressure at or near the airway of subject 12 with stochastic fluctuations in pressure. These stochastic fluctuations mimic similar oscillations present in so-called "bubble CPAP" systems. Since these stochastic fluctuations are intended to maintain the openness of the airway of subject 12, and not to drive ventilation, these fluctuations tend to have a higher frequency and/or lower magnitude than changes in pressure that drive ventilation. The magnitude (or mean or median magnitude) of the oscillations may be less than about 2 cm H₂O. In one embodiment, system 10 may include a pressure generator 14, a subject interface circuit 16, electronic storage 18, a user interface 20, one or more proximal or distal sensors 22, a pressure fluctuation valve 24, a processor 26, and/or other components.

The pressure generator 14 is configured to generate a pressurized flow of breathable gas for delivery to the airway of subject 12. The pressure generator 14 is configured to control one or more parameters of the pressurized flow of breathable gas to provide a therapeutic benefit to subject 12. The one or more parameters may include one or more of pressure, flow rate, gas composition, temperature, humidity, acceleration, velocity, acoustics, and/or other parameters. The pressure and/or flow rate of the pressurized flow of breathable gas are controlled by one or more components configured to pressurize and/or control the release of gas. For example, pressure generator 14 may include a pressure controlling valve, a bellows, a blower, an impeller, and/or other components that pressurize gas. To control the release of gas, pressure generator 14 may include one or more valves. The gas used by pressure generator 14 to generate the pressurized flow of breathable gas is obtained from one or more gas sources. The one or more gas sources may include one or more of a blower or compressor, a canister or tank, a Dewar, a wall gas source, ambient atmosphere, and/or other gas sources.

In one embodiment, pressure generator 14 is configured to obtain the gas used to generate the pressurized flow of breathable gas from a plurality of gas sources. In this embodiment, the relative concentrations of the gases obtained from the different gas sources may be controlled for therapeutic effect. For example, gas from ambient atmosphere may be used in a specific ratio with a purified oxygen gas to control increase the oxygen concentration of the pressurized flow of breathable gas.

The pressure generator 14 may be configured to generate the pressurized flow of breathable gas according to one or more modes. A non-limiting example of one such mode is Continuous Positive Airway Pressure (CPAP). CPAP has been used for many years and has proven to be helpful in promoting regular breathing. Another mode for generating the pressurized flow of breathable gas is Bi-level Positive Air Pressure (BiPAP®). In BiPAP®, two levels of positive air pressure (an inspiratory level and an expiratory level) are supplied to a subject. Generally, the timing of the inspiratory and expiratory pressure levels of pressure are controlled such that the inspiratory pressure level of positive air pressure is delivered to subject 12 during inhalation and the expiratory pressure level is delivered to subject 12 during exhalation. The timing of the inspiratory and expiratory pressure levels is coordinated to coincide with the breathing of subject 12 based on detection of gas parameters that indicate whether a user is currently inhaling or exhaling.

In one embodiment, pressure generator 14 is configured to generate the pressurized flow of breathable gas according to a mechanical ventilation mode. Unlike positive airway pressure therapy modes (such as CPAP and BiPAP®) designed to support the airway of subject 12 during spontaneous breathing, a mechanical ventilation mode is designed to mechanically ventilate subject 12. In this embodiment, the pressure of the pressurized flow of breathable gas is controlled to cause subject 12 to inhale and exhale as the pressure rises and falls (e.g., between an inspiratory level and an expiratory level).

It will be appreciated that the modes discussed above are not intended to be limiting. In one embodiment, pressure generator 14 may be configured to generate the pressurized flow of breathable gas according to a mode that provides noninvasive ventilation or invasive ventilation to subject 12.

The subject interface circuit 16 is configured to deliver the pressurized flow of breathable gas from pressure generator 14 to the airway of subject 12. In one embodiment, subject interface circuit 16 includes a conduit 28, an interface appliance 30, and/or other components. The conduit 28 conveys the pressurized flow of breathable gas to interface appliance 30, and interface appliance 30 delivers the pressurized flow of breathable gas to the airway of subject 12. In one embodiment, conduit 28 is formed from a flexible tubing. The conduit 28 may be sealed from ambient atmosphere, or conduit 28 may include one or more leaks *(e.g.,* leak valves) through which fluid within conduit 28 communicates with ambient atmosphere. Some examples of interface appliance 30 may include, for example, a nasal cannula, an endotracheal tube, a tracheotomy tube, a nasal mask, a nasal/oral mask, a full face mask, a total face mask, or other interface appliances that communication a flow of gas with an airway of a subject. The present invention is not limited to these examples, and contemplates delivery of the pressurized flow of breathable gas to subject 12 using any subject interface.

It will be appreciated that the illustration in FIG. 1 of subject interface circuit 16 as a single-limbed circuit is not intended to be limiting. The scope of this disclosure includes embodiments in which subject interface circuit 16 includes a second line that communicated with conduit 28 and/or interface appliance 30. The second line may be configured to exhaust exhaled gas from the airway of subject 12. Some of these embodiments are addressed below.

In one embodiment, electronic storage 18 comprises electronic storage media that electronically stores information. The electronic storage media of electronic storage 18 may include one or both of system storage that is provided integrally (i.e., substantially non-removable) with system 10 and/or removable storage that is removably connectable to system 10 via, for example, a port (e.g., a USB port, a firewire port, etc.) or a drive (e.g., a disk drive, etc.). Electronic storage 18 may include one or more of optically readable storage media (e.g., optical disks, etc.), magnetically readable storage media (e.g., magnetic tape, magnetic hard drive, floppy drive, etc.), electrical charge-based storage media (e.g., EEPROM, RAM, etc.), solid-state storage media (e.g., flash drive, etc.), and/or other electronically readable storage media. Electronic storage 18 may store software algorithms, information determined by processor 26, information received via user interface 20, and/or other information that enables system 10 to function properly. Electronic storage 18 may be (in whole or in part) a separate component within system 10, or electronic storage 18 may be provided (in whole or in part) integrally with one or more other components of system 10 (e.g., pressure generator 14, user interface 20, processor 26, *etc.).*

User interface 20 is configured to provide an interface between system 10 and subject 12 through which a user (*e.g*., subject 12, a caregiver, a therapy decision-maker, a researcher, *etc.*) may provide information to and receive information from system 10. This enables data, results, and/or instructions and any other communicable items, collectively referred to as "information," to be communicated between the user and one or more of pressure generator 14, electronic storage 18, pressure fluctuation valve 24, and/or processor 26. Examples of interface devices suitable for inclusion in user interface 20 include a keypad, buttons, switches, a keyboard, knobs, levers, a display screen, a touch screen, speakers, a microphone, an indicator light, an audible alarm, a printer, and/or other interface devices. In one embodiment, user interface 20 includes a plurality of separate interfaces. In one embodiment, user interface 20 includes at least one interface that is provided integrally with pressure generator 14, and a separate interface associated with pressure fluctuation valve 24.

It is to be understood that other communication techniques, either hard-wired or wireless, are also contemplated by the present invention as user interface 18. For example, the present invention contemplates that user interface 20 may be integrated with a removable storage interface provided by electronic storage 18. In this example, information may be loaded into system 10 from removable storage (e.g., a smart card, a flash drive, a removable disk, etc.) that enables the user(s) to customize the implementation of system 10. Other exemplary input devices and techniques adapted for use with system 10 as user interface 20 include, but are not limited to, an RS-232 port, RF link, an IR link, modem (telephone, cable or other). In short, any technique for communicating information with system 10 is contemplated by the present invention as user interface 20.

The sensor 22 is configured to generate one or more output signals conveying information related to one or more gas parameters of the gas breathed by subject 12. The one or more parameters may include, for example, one or more of a flow rate, a volume, a pressure, a composition (*e.g*., concentration(s) of one or more constituents), humidity, temperature, acceleration, velocity, acoustics, changes in a parameter indicative of respiration, and/or other gas parameters. Although FIG. 1 depicts sensor 22 as being located at or near interface appliance 30, this is not intended to be limiting. The sensor 22 may include one or more sensors monitoring gas parameters within interface appliance 30, conduit 28, pressure generator 14, and/or elsewhere between the generation of the pressurized flow of breathable gas and the airway of subject 12.

The pressure fluctuation valve 24 is configured to create stochastic fluctuations in pressure of the pressurized flow of breathable gas at or near the airway of subject 12. The pressure fluctuation valve 24 is configured such that the stochastic fluctuations mimic oscillations in pressure that would be present in a bubble CPAP system. That is, if the pressurized flow of breathable gas were passed through a water container, the oscillations in pressure caused by the water container would be similar in frequency, magnitude, timing, and/or randomness to the stochastic fluctuations in pressure caused by pressure fluctuation valve 24. As used herein, "stochastic" refers to the non-deterministic nature of the fluctuations in pressure. This means that one or more parameters of the fluctuations (*e.g*., frequency, individual timing, magnitude, direction, *etc.)* are random, pseudo-random, probabilistic, and/or otherwise non-deterministic.

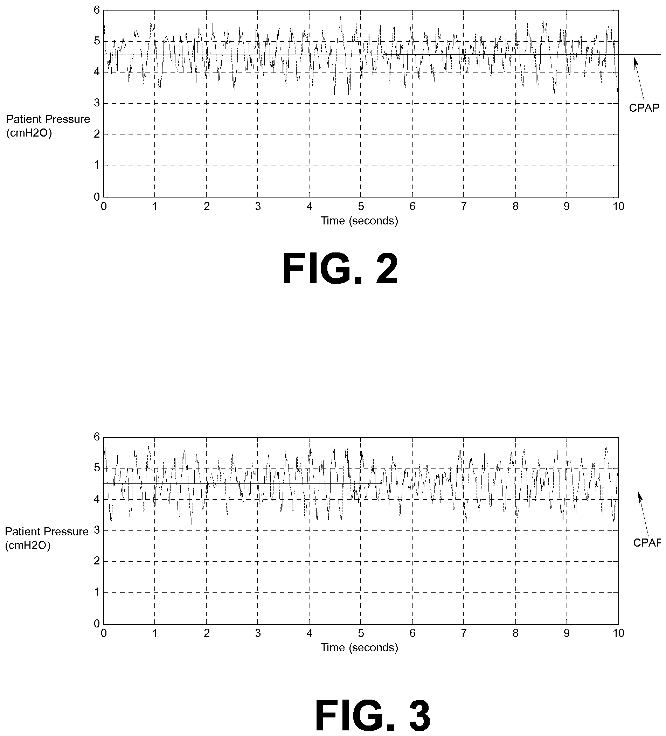

By way of illustration, FIG. 2 includes a plot of pressure at or near the airway of a subject in a conventional bubble CPAP system. As can be seen in FIG. 2, passing the pressurized flow of breathable gas through a water container downstream from the subject causes oscillations in pressure about a mean or median pressure level provided by a pressure generator associated with the conventional bubble CPAP system.

FIG. 3 shows a plot of pressure at or near the airway of a subject in a system implementing a pressure fluctuation valve similar to or the same as pressure fluctuation valve 24 (shown in FIGS. 1 and 5-8, and described herein). As can be seen in FIG. 3, the pressure fluctuation valve causes stochastic fluctuations that mimic the pressure oscillations of a bubble CPAP system. To mimic the oscillations of a bubble CPAP system, the fluctuations are stochastic (*e.g*., random or pseudo-random), and have magnitudes that are similar to the oscillations of the bubble CPAP system. Further, the frequency of the stochastic fluctuations is similar to the frequency of oscillations found in the bubble CPAP system.

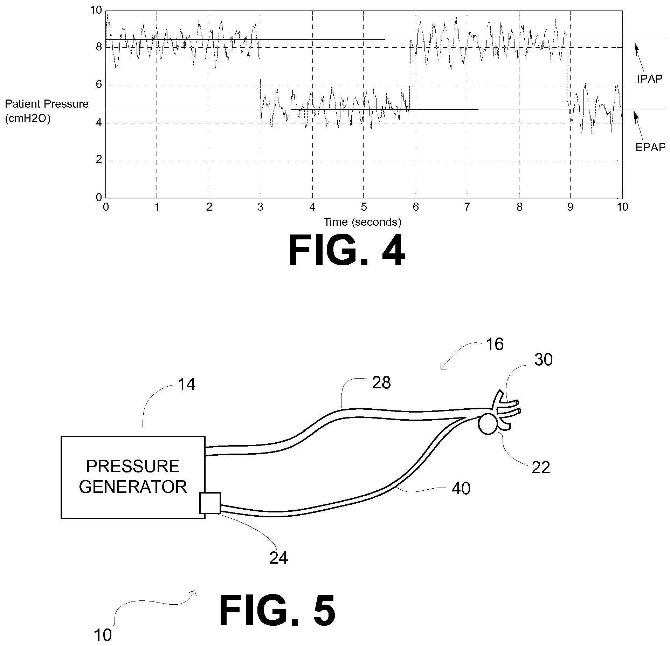

FIG. 4 shows a plot of pressure at or near the airway of a subject in a system implementing a pressure fluctuation valve similar to or the same as pressure fluctuation valve 24 (shown in FIGS. 1 and 5-8, and described herein). In the embodiment illustrated in FIG. 4, the pressurized flow of breathable gas is being delivered to the subject in a bi-level mode of therapy (*e.g.,* BiPAP®). As such, the pressurized flow of breathable gas is generated such that pressure at the airway of the subject oscillates between an inspiratory pressure level during inhalation and an expiratory pressure during exhalation. In this embodiment, the pressure fluctuation valve is configured such that the stochastic fluctuations are superimposed on top of the oscillations between the inspiratory pressure level and the expiratory pressure level.

Returning to FIG. 1, the scope of this disclosure contemplates any mechanical element capable of creating fluctuations in pressure at or near the airway of subject 12 as pressure fluctuation valve 24. The stochastic nature of the fluctuations may be caused by the mechanical configuration of pressure fluctuation valve 24, and/or may be caused by control of pressure fluctuation valve 24 in a stochastic manner. Some specific, though non-limiting, embodiments of pressure fluctuation valve 24 are described below.

In one embodiment, one or more parameters of the stochastic fluctuations are configurable by a user. For example, the user interface 20 may include an interface configured to receive user selections that configure the one or more parameters. The one or more parameters may include, a range of frequencies of the stochastic fluctuations, a mean or median frequency of the stochastic fluctuations, a range of magnitudes of the stochastic fluctuations, a mean or median magnitude of the stochastic fluctuations, a range of pressure levels within which the stochastic fluctuations occur, a maximum deviation away from the mean pressure level, and/or other parameters.

In one embodiment, pressure fluctuation valve 24 is included integrally in a common device with one or more of pressure generator 14, conduit 28 and/or interface appliance 30. In one embodiment, pressure fluctuation valve 24 is a separate component that is selectively inserted to system 10 (*e.g*., between pressure generator 14 and conduit 28, within conduit 28, between conduit 28 and interface appliance 30, within interface appliance 30, downstream from interface appliance 30, and/or at other locations in system 10). In an embodiment in which pressure fluctuation valve 24 is included integrally in a common device with pressure generator 14, pressure fluctuation valve 24 may be a component in pressure generator 14 that pressurizes the pressurized flow of breathable gas *(e.g.,* alone or in conjunction with other components of pressure generator 14).

The provision of pressure fluctuation valve 24 with system 10, integrally with the other components or as a separate component, may provide various enhancements over existing bubble CPAP systems. For example, system 10 maybe a fully functional ventilation and/or positive airway pressure therapy system (*e.g*., including pressure generator 14, sensor 22, and/or processor 26). Such systems tend to be far more refined and/or sophisticated than conventional bubble CPAP systems. For example, system 10 may by configurable to operate in accordance with a wider variety of therapy modes (*e.g*., positive airway pressure therapy, mechanical ventilation, and/or other modes) and/or pressure settings than conventional bubble CPAP systems. The system 10 may provide for the electronic monitoring (*e.g*., based on the output signals generated by sensor 22) of breathing parameters of subject 12 *(e.g.,* respiratory rate, pressure, capnometry, tidal volume, FiO₂, *etc*.). This is a more precise and comprehensive monitoring than can be accomplished via a conventional bubble CPAP system. Based on this monitoring, therapy modes, alarms, and/or shutoffs not implemented in conventional CPAP systems may be implemented. Further, the monitoring of such breathing parameters may be implemented by a caregiver or therapy decision-maker in determining the effectiveness of the therapy and/or modifications to the therapy, whereas such information may not be available if a conventional CPAP system is implemented. The system 10 may be configured to deliver oxygen enriched gas in the pressurized flow of breathable gas to subject 12 in response to the monitored parameters.

Processor 26 is configured to provide information processing capabilities in system 10. As such, processor 26 may include one or more of a digital processor, an analog processor, a digital circuit designed to process information, an analog circuit designed to process information, a state machine, and/or other mechanisms for electronically processing information. Although processor 26 is shown in FIG. 1 as a single entity, this is for illustrative purposes only. In some implementations, processor 26 may include a plurality of processing units. These processing units may be physically located within the same device, or processor 26 may represent processing functionality of a plurality of devices operating in coordination. For example, in one embodiment, some of the functionality attributed below to processor 26 is provided by one or more components included in a device with pressure generator 14, while other functionality attributed below to processor 26 is provided by one or more components included in a separate device with pressure fluctuation valve 24.

As is shown in FIG. 1, processor 26 may be configured to execute one or more computer program modules. The one or more computer program modules may include one or more of a gas parameter module 32, a control module 34, a breathing parameter module 36, a fluctuation module 38, and/or other modules. Processor 26 may be configured to execute modules 32, 34, 36, and/or 38 by software; hardware; firmware; some combination of software, hardware, and/or firmware; and/or other mechanisms for configuring processing capabilities on processor 26.

It should be appreciated that although modules 32, 34, 36, and 38 are illustrated in FIG. 1 as being co-located within a single processing unit, in implementations in which processor 26 includes multiple processing units, one or more of modules 32, 34, 36, and/or 38 may be located remotely from the other modules. The description of the functionality provided by the different modules 32, 34, 36, and/or 38 described below is for illustrative purposes, and is not intended to be limiting, as any of modules 32, 34, 36, and/or 38 may provide more or less functionality than is described. For example, one or more of modules 32, 34, 36, and/or 38 may be eliminated, and some or all of its functionality may be provided by other ones of modules 32, 34, 36, and/or 38. As another example, processor 26 may be configured to execute one or more additional modules that may perform some or all of the functionality attributed below to one of modules 32, 34, 36, and/or 38.

The gas parameter module 32 is configured to determine information related to one or more gas parameters of the gas within conduit 28 and/or interface appliance 30 *(e.g.,* the pressurized flow of breathable gas). The one or more gas parameters are determined based on the output signals of sensor 22. The one or more gas parameters may include one or more of a pressure, a flow rate, a peak flow, a composition, a humidity, a temperature, an acceleration, a velocity, a thermal energy dissipated (*e.g.,* in a mass flowmeter, *etc*.), and/or other gas parameters. The parameter(s) determined by gas parameter module 32 may be presented to a user (*e.g*., through user interface 20), and/or used as a triggering parameter for an alarm, for a shutoff, and/or for other functions.

Control module 34 is configured to control pressure generator 14. Controlling pressure generator 14 includes adjusting one or more of the parameters of the pressurized flow of breathable gas. The control module 34 may control pressure generator 14 to adjust the one or more parameters of the pressurized flow of breathable gas in accordance with a therapy mode, to relieve excess pressure or flow being delivered to subject 12, and/or for other reasons.

The breathing parameter module 36 is configured to determine one or more breathing parameters of the respiration of subject 12. The breathing parameter module 36 may determine the one or more breathing parameters based on the one or more gas parameters determined by gas parameter module 32 and/or from the output signals generated by sensor 22. For example, the one or more breathing parameters may include one or more of a respiration rate, an inhalation flow rate, an inhalation period, an exhalation flow rate, an exhalation period, a tidal volume, a breathing rate, a breath period, a peak flow, a flow curve shape, a pressure curve shape, expiration-to-inspiration transitions, inspiration-to-expiration transitions, fraction of inspired oxygen, and/or other breathing parameters. The parameter(s) determined by breathing parameter module 36 may be used as a triggering parameter for an alarm, for a shutoff, and/or for other functionality.

The fluctuation module 38 is configured to control operation of pressure fluctuation valve 24. Controlling the operation of pressure fluctuation valve 24 include beginning and/or ending the stochastic fluctuations, controlling the operation of pressure fluctuation valve 24 to define or set one or more of a range of frequencies of the stochastic fluctuations, a mean or median frequency of the stochastic fluctuations, a range of magnitudes of the stochastic fluctuations, a mean or median magnitude of the stochastic fluctuations, a range of pressure levels within which the stochastic fluctuations occur, a maximum deviation away from the mean pressure level, and/or other parameters. In one embodiment, fluctuation module 38 controls the operation of pressure fluctuation valve 24 in accordance with user selections (*e.g*., as received through user interface 20).

FIG. 5 illustrates an embodiment of system 10 including an exhaust limb formed by an exhaust conduit 40 included in subject interface circuit 16. The exhaust conduit 40 configured to receive gas exhausted from conduit 28 and/or interface appliance 30, including gas that has been exhaled by subject 12 into interface appliance 30. In the embodiment shown in FIG. 5, exhaust conduit 40 communicates the gas back to pressure generator 14. However, this is not intended to be limiting. The exhaust conduit 40 may exhaust the gas to a separate device, and/or to ambient atmosphere without returning the gas to pressure generator 14.

In the embodiment of FIG. 5, the pressure fluctuation valve 24 is disposed in system 10 to receive gas within exhaust conduit 40. By disrupting the flow of gas through exhaust conduit 40, pressure fluctuation valve 24 effectively alters the level of pressure within interface appliance 30 to cause the stochastic fluctuations in pressure at or near the airway of subject 12. The primary difference between this configuration and the configuration shown in FIG. 1 is that pressure fluctuation valve 24 is "downstream" from subject 12, rather than "upstream." It will be appreciated that the depiction of pressure fluctuation valve 24 downstream from subject 12 in the dual-limb system shown in FIG. 5 is not intended to be limiting. In a dual-limb system, pressure fluctuation valve 24 may still be located upstream from subject 12 (*e.g.,* as shown in the single-limb system of FIG. 1) without departing from the scope of this disclosure. Further, the depiction of pressure fluctuation valve 24 being disposed between exhaust conduit 40 and subject 12 is not intended to be limiting. The pressure fluctuation valve 24 may be disposed in exhaust limb at an exhaust port on interface appliance 30, between interface appliance 30 and exhaust conduit 40, within exhaust conduit 40, and/or internally in pressure generator 14.

Referring back to FIG. 1, in one embodiment, stochastic fluctuations in pressure are enhanced by a component of pressure generator 14 other than pressure fluctuation valve 24. For example, a blower or bellows associated with pressure generator 14 may be controlled to create or enhance the stochastic fluctuations in pressure.

In order to accomplish such control of the blower or bellows (or other component of pressure generator 14), the fluctuation module 38 is configured to introduce stochastic fluctuations into the operation of pressure generator 14 that causes the pressurized flow of breathable gas pressure generator 14 to experience stochastic fluctuations in pressure. Fluctuation module 38 may introduce the fluctuations into, for example the speed of a blower associated with pressure generator 14, a current provided to a motor associated with pressure generator 14, and/or may introduce fluctuations into other aspects of the operation of pressure generator 14. The parameters of the fluctuations may be determined in a random or pseudo-random manner by fluctuation module. For example, the magnitude, direction, frequency, timing, and/or other parameters of the fluctuations may be determined randomly or pseudo-randomly. Bounds, limits, or other constraints on these magnitudes may be obtained from user selection.

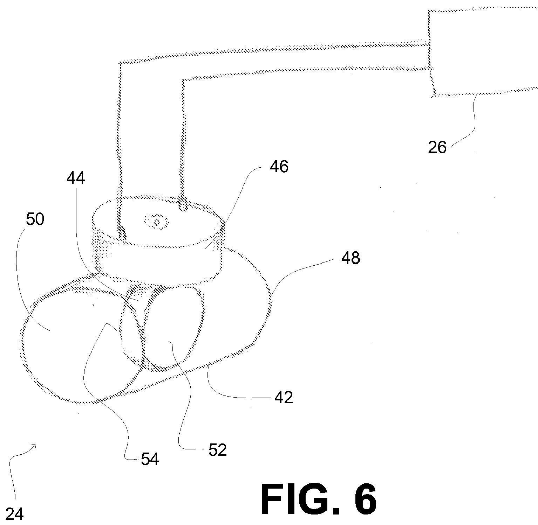

FIG. 6 illustrates an embodiment of pressure fluctuation valve 24. In the embodiment shown in FIG. 6, pressure fluctuation valve 24 includes a conduit 42, a diaphragm 44, a motor 46, and/or other components.

The conduit 42 includes a first end 48 and a second end 50. The conduit 42 forms a flow path between first end 48 and second end 50. During use, pressure fluctuation valve 24 is installed within a system configured to provide a pressurized flow of breathable gas to the airway of a subject such that the pressurized flow of breathable gas passes through the flow path between first end 48 and second end 50 (*e.g.,* upstream or downstream from the subject).

The diaphragm 44 is formed as a thin member having a first surface 52 and a second surface 54. The diaphragm 44 is mounted pivotally to the inner surface of conduit 42 at an interface of diaphragm 44 with motor 46. The shape of first surface 52 and/or second surface 54 corresponds to the inner cross sections of conduit 42 such that as diaphragm 44 rotates about the pivotal engagement with motor 46 the first surface 52 and/or second surface 54 at least partially block the flow of gas through conduit 42. However, due to the thinness of diaphragm 44, gas is allowed to flow through conduit 42 relatively unimpeded when diaphragm 44 first surface 52 and second surface 54 face the sidewalls of conduit 42. In one embodiment, the diaphragm 44 is formed from a resiliently flexible material. In one embodiment, the flexibility of diaphragm 44 is controllable by running an electrical current through diaphragm 44.

The motor 46 is configured to rotate diaphragm 44 within conduit 42. This causes the diaphragm 44 to disrupt the flow of gas through conduit 42 in such a manner that causes stochastic fluctuations in pressure at or near the airway of the subject. More specifically, as diaphragm 44 rotates within conduit 42, first surface 52 and/or second surface 54 blocks more or less gas (depending on its position), thereby causing the stochastic fluctuations. It will be appreciated that the "rotation" of diaphragm 44 is not necessarily full rotations about an axis. Instead, the "rotation" of diaphragm 44 may refer to oscillations back and forth in different rotational directions.

In one embodiment, the flexibility of diaphragm 44 contributes to the stochastic nature of the fluctuations, as diaphragm 44 is flexed by the flow of gas through conduit 42. In one embodiment, the one or more aspects of the rotation of diaphragm 44 within conduit 42 are stochastic, which contributes to the stochastic nature of the fluctuations. For example, the axis of rotation, the rate of rotation, the rotational acceleration, the positions at which direction of rotation is changed, and/or other aspects of the rotation of diaphragm 44 may be varied stochastically (e.g., through control and/or structure of motor 46).

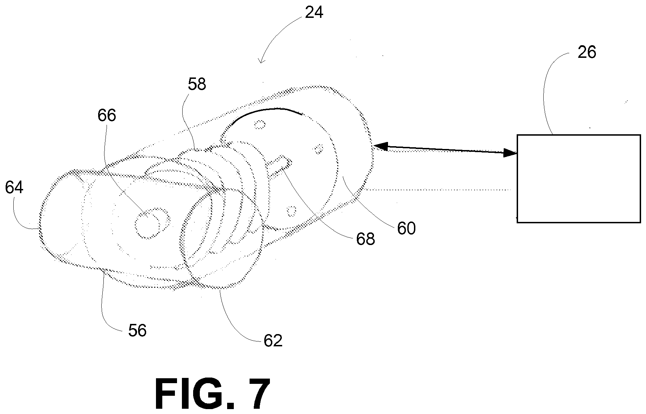

FIG. 7 illustrates an embodiment of pressure fluctuation valve 24. In the embodiment shown in FIG. 7, pressure fluctuation valve 24 includes a conduit 56, a bellows 58, a motor 60, and/or other components.

The conduit 56 includes a first end 62 and a second end 64. The conduit 56 forms a flow path between first end 62 and second end 64. During use, pressure fluctuation valve 24 is installed within a system configured to provide a pressurized flow of breathable gas to the airway of a subject such that the pressurized flow of breathable gas passes through the flow path between first end 62 and second end 64 (*e.g*., upstream or downstream from the subject).

The bellows 58 is configured to output gas from an output 66. The bellows 58 is expanded and contracted to take in (through an input not shown) and output (through output 66) gas by longitudinal motion of an end of bellows 58 opposite output 66 along a shaft 68. The output of gas into conduit 56 through output 66 tends to disrupt the flow of gas through conduit 56, thereby causing fluctuations of pressure at or near the airway of the subject.

The motor 60 is configured to drive the end of bellows 58 opposite from output 66 back and forth along shaft 68. In one embodiment, motor 60 includes a voice coil that creates a magnetic field providing the motive force that drives the end of bellows 58 back and forth along shaft 68. The imprecise nature of the bellows 58 and/or the interaction of the gas output from bellows 58 with the gas in conduit 56 may cause the fluctuations of pressure at or near the airway of the subject caused by pressure fluctuation valve 24 to be stochastic. In one embodiment, the electrical current provided to drive motor 60 is varied randomly or pseudo-randomly. This may contribute to the stochastic nature of the fluctuations in pressure at or near the airway of the subject.

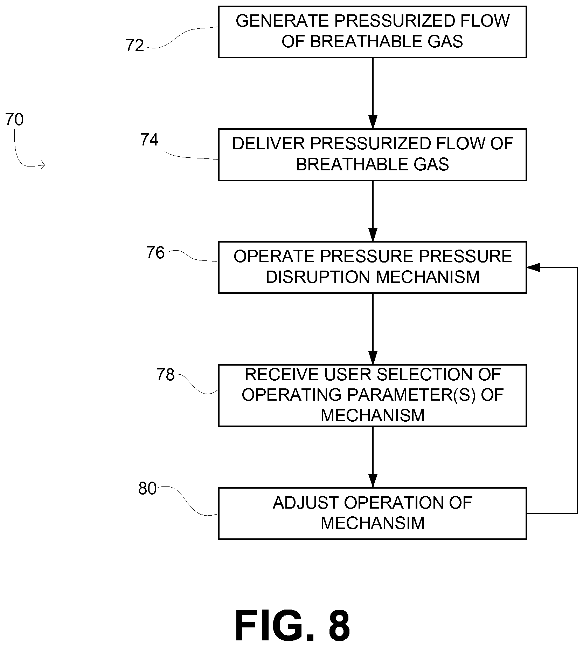

FIG. 8 illustrates a method 70 of providing a pressurized flow of breathable gas to the airway of a subject. The operations of method 70 presented below are intended to be illustrative. In some embodiments, method 70 may be accomplished with one or more additional operations not described, and/or without one or more of the operations discussed. Additionally, the order in which the operations of method 70 are illustrated in FIG. 8 and described below is not intended to be limiting.

At an operation 72, a pressurized flow of breathable gas is generated. One or more gas parameters of the pressurized flow of breathable gas are controlled to provide therapeutic benefit to the subject. In one embodiment, operation 72 is performed by a pressure generator similar to or the same as pressure generator 14 (shown in FIG. 1 and described above).

At an operation 74, the pressurized flow of breathable gas is delivered to the airway of the subject. In one embodiment, operation 74 is performed by a subject interface circuit similar to or the same as subject interface circuit 16 (shown in FIG. 1 and described above).

At an operation 76, a pressure fluctuation valve and/or the pressure generator are operated to create stochastic fluctuations in pressure of the pressurized flow of breathable gas at or near the airway of the subject. The pressure fluctuation valve may be similar to or the same as pressure fluctuation valve 24 (shown in FIGS. 1 and 5-7, and described above).

At an operation 78, user selection of one or more parameters for the stochastic fluctuations is received. The one or more parameters may include one or more of, for example, a range of frequencies of the stochastic fluctuations, a mean or median frequency of the stochastic fluctuations, a range of magnitudes of the stochastic fluctuations, a mean or median magnitude of the stochastic fluctuations, a range of pressure levels within which the stochastic fluctuations occur, a maximum deviation away from the mean pressure level, and/or other parameters. In one embodiment, operation 78 is performed by a user interface similar to or the same as user interface 20 (shown in FIG. 1 and described above).

At an operation 80, operation of the pressure fluctuation valve and/or the pressure generator is adjusted in accordance with the received user selection. In one embodiment, operation 80 is performed by a fluctuation module similar to or the same as fluctuation module 38 (shown in FIG. 1 and described above).

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A system (10) configured to provide a pressurized flow of breathable gas to the airway of a subject (12), the system (10) comprising:
means for generating (14) the pressurized flow of breathable gas such that one or more gas parameters of the pressurized flow of breathable gas provide a therapeutic benefit to the subject (12);
means for delivering (16) the pressurized flow of breathable gas to the airway of the subject; and
means for creating stochastic fluctuations (24,26,38) in pressure of the pressurized flow of breathable gas at or near the airway of the subject (12),
**characterized in that** the means for creating stochastic fluctuations (24,26,38) are further configured such that the stochastic fluctuations in pressure mimic oscillations in pressure that would be caused by passing gas provided to and/or received from the airway of the subject through a water container;
**in that** the means for creating stochastic fluctuations comprises a processor (26), a fluctuation module (38) executable via the processor (26), and a pressure fluctuation valve (24); and
**in that** the fluctuation module (38) is configured to control the pressure fluctuation valve (24) in a stochastic manner to (a) to begin and/or end the stochastic fluctuations and (b) define or set one or more of a range of frequencies of the stochastic fluctuations, a mean or median frequency of the stochastic fluctuations, a range of magnitudes of the stochastic fluctuations, a mean or median magnitude of the stochastic fluctuations, a range of pressure levels within which the stochastic fluctuations occur, and a maximum deviation away from a mean pressure level.

2. The system (10) of claim 1, wherein the means for creating stochastic fluctuations (24,26,38) are provided within a single integrated device along with the means for generating (14) the pressurized flow of breathable gas.

3. The system (10) of claim 1 or 2, further comprising means for receiving (20) user selection of a range of frequencies for the stochastic fluctuations, a range of magnitudes for the stochastic fluctuations, and/or a range of pressures within which the fluctuations occur.

4. The system (10) of any of claims 1 to 3, wherein the means for generating (14) the pressurized flow of breathable gas is configured such that pressure at the airway of the subject (12) oscillates between an inspiratory pressure level during inhalation by the subject and an expiratory pressure level during exhalation by the subject, and wherein the means for creating stochastic fluctuations (24,26,38) are configured such that the stochastic fluctuations are superimposed on top of the oscillations of pressure between the inspiratory pressure level and the expiratory pressure level.

5. The system (10) of any of claims 1 to 4, wherein the range of magnitudes of the stochastic fluctuations, or the mean or median magnitude of the stochastic fluctuations is set or defined to be less than 2cm H₂O.

6. The system (10) of any of claims 1 to 5,
wherein the means for generating the pressurized flow of breathable gas is a pressure generator (14);
wherein the means for delivering the pressurized flow of breathable gas to the airway of the subject (12) is a subject interface circuit (16); and
wherein the means for creating stochastic fluctuations is a pressure fluctuation mechanism (24,26,38).

7. The system (10) of claim 6, wherein the pressure fluctuation mechanism (24,26,38) further comprises a component of the pressure generator (14).

## Patentansprüche

1. System (10), konfiguriert für die Zuführung eines unter Druck stehenden Stroms von Atemgas in die Atemwege einer Person (12), wobei das System (10) umfasst:
Mittel zum Generieren (14) des unter Druck stehenden Stroms von Atemgas, so dass einer oder mehr Gasparameter des unter Druck stehenden Stroms von Atemgas einen therapeutischen Nutzen für die Person (12) bereitstellen;
Mittel zum Ausliefern (16) des unter Druck stehenden Stroms von Atemgas in die Atemwege der Person; und
Mittel zum Erzeugen stochastischer Druckschwankungen (24,26,38) des unter Druck stehenden Stroms von Atemgas an oder in der Nähe der Atemwege der Person (12),
**dadurch gekennzeichnet, dass** die Mittel zum Erzeugen stochastischer Schwankungen (24,26,38) ferner so konfiguriert sind, dass die stochastischen Druckschwankungen Druckschwingungen nachahmen, die durch das vorbeifließende Gas verursacht würden, das durch einen Wasserbehälter für die Atemwege der Person bereitgestellt bzw. von ihnen aufgenommen wird;
dass die Mittel zum Erzeugen stochastischer Schwankungen einen Prozessor (26), ein über den Prozessor (26) ausführbares Fluktuationsmodul (38) und ein Druckfluktuationsventil (24) umfassen; und
dass das Fluktuationsmodul (38) dazu konfiguriert ist, das Druckfluktuationsventil (24) in stochastischer Weise zu steuern, um (a) die stochastischen Schwankungen zu starten bzw. zu beenden und (b) einen oder mehr Frequenzbereiche der stochastischen Schwankungen, eine durchschnittliche oder mediane Frequenz der stochastischen Schwankungen, einen Größenbereich der stochastischen Schwankungen, eine durchschnittliche oder mediane Größenordnung der stochastischen Schwankungen, einen Druckpegelbereich, innerhalb dessen die stochastischen Schwankungen auftreten, und eine Höchstabweichung von einem durchschnittlichen Druckpegel zu definieren oder festzulegen.

2. System (10) nach Anspruch 1, wobei die Mittel zum Erzeugen stochastischer Schwankungen (24,26,38) von einem einzigen integrierten Gerät gleichzeitig mit den Mitteln zum Generieren (14) des unter Druck stehenden Stroms von Atemgas bereitgestellt werden.

3. System (10) nach Anspruch 1 oder 2, ferner umfassend Mittel zum Empfangen (20) einer Benutzerauswahl aus einem Frequenzbereich für die stochastischen Schwankungen, eines Größenbereichs für die stochastischen Schwankungen, bzw. eines Druckbereichs innerhalb dessen die Schwankungen auftreten.

4. System (10) nach einem beliebigen der Ansprüche 1 bis 3, wobei das Mittel zum Generieren (14) des unter Druck stehenden Stroms von Atemgas so konfiguriert ist, dass der Druck an den Atemwegen der Person (12) zwischen einem Inspirationsdruckpegel beim Einatmen der Person und einem Exspirationsdruckpegel beim Ausatmen der Person schwingt, und wobei die Mittel zum Erzeugen stochastischer Schwankungen (24,26,38) so konfiguriert sind, dass die stochastischen Schwankungen die Druckschwingungen zwischen dem Inspirationsdruckpegel und dem Exspirationsdruckpegel überlagern.

5. System (10) nach einem beliebigen der Ansprüche 1 bis 4, wobei der Größenbereich der stochastischen Schwankungen, oder die durchschnittliche oder mediane Größenordnung der stochastischen Schwankungen so definiert oder festgelegt ist, dass sie unterhalb von 2 cm H₂O liegt.

6. System (10) nach einem beliebigen der Ansprüche 1 bis 5,
wobei das Mittel zum Generieren des unter Druck stehenden Stroms von Atemgas ein Druckgenerator (14) ist;
wobei das Mittel zum Ausliefern des unter Druck stehenden Stroms von Atemgas zu den Atemwegen der Person (12) ein Schnittstellenkreis der Person (16) ist; und
wobei das Mittel zum Erzeugen stochastischer Schwankungen ein Druckfluktuationsmechanismus (24,26,38) ist.

7. Das System (10) nach Anspruch 6, wobei der Druckfluktuationsmechanismus (24,26,38) ferner eine Komponente des Druckgenerators (14) umfasst.

## Revendications

1. Système (10) configuré pour amener un flux de gaz respiratoire sous pression aux voies respiratoires d'un sujet (12), le système (10) comprenant :
des moyens pour générer (14) le flux de gaz respiratoire sous pression de telle sorte qu'un ou plusieurs paramètres de gaz du flux de gaz respiratoire sous pression présentent un bénéfice thérapeutique pour le sujet (12) ;
des moyens pour délivrer (16) le flux de gaz respiratoire sous pression aux voies respiratoires du sujet ; et
des moyens pour créer des fluctuations stochastiques (24, 26, 38) dans la pression du flux de gaz respiratoire au niveau ou à proximité des voies respiratoires du sujet (12),
**caractérisé en ce que** les moyens pour créer des fluctuations stochastiques (24, 26, 38) sont en outre configurés de telle sorte que les fluctuations stochastiques de pression imitent les oscillations de pression qui seraient causées par le passage du gaz amené vers les voies aériennes et/ou reçu de celles-ci à travers un récipient d'eau ;
**en ce que** les moyens pour créer des fluctuations stochastiques comprennent un processeur (26), un module de fluctuation (38) exécutable via le processeur (26), et une vanne de fluctuation de pression (24) ; et
**en ce que** le module de fluctuation (38) est configuré pour contrôler la valve de fluctuation de pression (24) d'une manière stochastique pour (a) débuter les fluctuations stochastiques et/ou y mettre fin et (b) définir ou régler une ou plusieurs d'une plage de fréquences des fluctuations stochastiques, d'une fréquence moyenne ou médiane des fluctuations stochastiques, d'une plage de grandeurs des fluctuations stochastiques, d'une grandeur moyenne ou médiane des fluctuations stochastiques, d'une plage de niveaux de pression à l'intérieur de laquelle se produisent les fluctuations stochastiques, et d'une déviation maximale par rapport à un niveau de pression moyen.

2. Système (10) selon la revendication 1, dans lequel les moyens pour créer des fluctuations stochastiques (24, 26, 38) sont prévus à l'intérieur d'un seul dispositif intégré en conjugaison avec les moyens pour générer (14) le flux de gaz respiratoire sous pression.

3. Système (10) selon la revendication 1 ou 2, comprenant en outre des moyens pour recevoir (20) une sélection utilisateur d'une plage de fréquences pour les fluctuations stochastiques, d'une plage de grandeurs pour les fluctuations stochastiques, et/ou d'une plage de pressions à l'intérieur de laquelle se produisent les fluctuations.

4. Système (10) selon l'une quelconque des revendications 1 à 3, dans lequel les moyens pour générer (14) le flux de gaz respiratoire sous pression sont configurés de telle sorte que la pression au niveau des voies aériennes du sujet (12) oscille entre un niveau de pression inspiratoire lors de l'inhalation par le sujet et un niveau de pression expiratoire lors de l'exhalation par le sujet, et dans lequel les moyens pour créer des fluctuations stochastiques (24, 26, 38) sont configurés de telle sorte que les fluctuations stochastiques soient superposées aux oscillations de pression entre le niveau de pression inspiratoire et le niveau de pression expiratoire.

5. Système (10) selon l'une quelconque des revendications 1 à 4, dans lequel la plage de grandeurs des fluctuations stochastiques, ou la grandeur moyenne ou médiane des fluctuations stochastiques est réglée ou définie comme étant inférieure à 2 cm H₂O.

6. Système (10) selon l'une quelconque des revendications 1 à 5,
dans lequel les moyens pour générer le flux de gaz respiratoire sous pression sont un générateur de pression (14) ;
dans lequel les moyens pour délivrer le flux de gaz respiratoire sous pression aux voies respiratoires du sujet (12) sont un circuit d'interface sujet (16) ; et
dans lequel les moyens pour créer des fluctuations stochastiques sont un mécanisme de fluctuation de pression (24, 26, 38).

7. Système (10) selon la revendication 6, dans lequel le mécanisme de fluctuation de pression (24, 26, 38) comprend en outre un composant du générateur de pression (14).
